(19) Europäisches Patentamt
European Patent Office
Office européen des brevets

(11) Publication number: **0 201 239**
**B1**

(12) **EUROPEAN PATENT SPECIFICATION**

(45) Date of publication of patent specification: **18.10.89**

(21) Application number: **86303039.1**

(22) Date of filing: **22.04.86**

(51) Int. Cl.[4]: **C 12 N 15/00,** C 12 C 11/00, C 12 P 7/06, C 12 P 21/02 // C12N9/38

(54) **Process for the isolation of fermentation products.**

(30) Priority: **22.04.85 GB 8510219**

(43) Date of publication of application: **12.11.86 Bulletin 86/46**

(45) Publication of the grant of the patent: **18.10.89 Bulletin 89/42**

(84) Designated Contracting States: **AT BE CH DE FR IT LI LU NL SE**

(56) References cited:
**EP-A-0 079 739**
**EP-A-0 147 198**

**CHEMICAL ABSTRACTS, vol. 102, no. 19; 13th May 1985, page 135, abstract no. 161231r, Columbus, Ohio, US; R.C.A. Henderson et al.; "The transformation of brewing yeasts with a plasmid containing the gene for copper resistance"**

**BREWERS' GUARDIAN; September 1984, pages 34-37; R.S. TUBB: "Genetic development of yeast strains"**

(73) Proprietor: **Delta Biotechnology Limited
137 High Street
Burton on Trent DE14 1JZ (GB)**

(72) Inventor: **Hinchliffe, Edward
44 Hall Road Rolleston-on-Dove
Burton on Trent East Staffordshire (GB)**
Inventor: **Kenny, Enda
11 Rouen Way
Ashby-de-la-Zouch Leicestershire (GB)**

(74) Representative: **Bassett, Richard Simon et al
ERIC POTTER & CLARKSON 14 Oxford Street
Nottingham NG1 5BP (GB)**

(56) References cited:
**PROC. NATL. ACAD. SCI., vol. 79, December 1982, pages 7410-7414: L. GUARENTE et al.: " A GAL10-CYC1 hybrid yeast promoter identifies the GAL4 regulatory region as an upstream site"**

**EP 0 201 239 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the isolation of fermentation products.

In the manufacture of alcohol by fermentation, sugars in aqueous solution are converted into ethanol by fermentation with yeast. The yeast grows during the fermentation and although a small proportion of the yeast may be used in a subsequent fermentation process, the remainder of the yeast constitutes an excess that must be disposed of. While this excess yeast has some uses, e.g., in animal feedstuffs and the manufacture of yeast extracts, the quantity of excess yeast produced is large and its market value is relatively low.

Large scale fermentations of this kind fall into three broad categories:

(1) Fermentations in which the fermented aqueous medium obtained is the desired end product. Into this category fall ordinary brewing processes for the production of beer (a term which, as used herein, includes ales, stouts, lagers and other fermented drinks based on malt), cider and other fermented drinks.

(2) Fermentations in which the desired end product is a distilled drinkable alcoholic concentrate. Into this category fall fermentations for production of whiskies, brandies and other spirits, and alcohol for use in fortifying other drinks.

(3) Fermentations for the production of alcohol for industrial use. Into this category fall fermentations carried out in some countries on a large scale for the production of fuel alcohol, and fermentations of certain industrial effluents, e.g. from paper pulp manufacture. The production of excess yeast is a characteristic of all these industrial processes.

Considerable interest has been shown in recent years in the genetic modification of microorganisms so that they become able to produce heterologous proteins and peptides, that is to say proteins and peptides which are not produced by their natural genetic constituents. A variety of microrganisms have been used for such genetic manipulation, and, amongst these, yeast have attracted a certain amount of interest. However, yeasts used in laboratory experiments are not normally the same as the yeasts used in large scale industrial fermentations involving the production of alcohol, and the conditions of growth of yeast in the laboratory are very different from those encountered by yeasts in an industrial alcoholic fermentation.

The yeasts are a group of lower eukaryotic micro-organisms showing biological and biochemical diversity. In common usage the term "yeast" is used to describe strains of *Saccharomyces cerevisiae* that have commercial value in baking, brewing and distilling. Related yeasts are used in wine making and sake brewing, as well as in the production of fuel alcohol from sucrose or hydrolysed starch, and in the treatment of effluents.

All the yeasts used for brewing, baking and distilling may be taxonomically classified as *Saccharomyces cerevisiae*. Included within this classification are the top fermenting ale yeasts (*S. cerevisiae*) and the bottom-fermenting lager yeats (*S. uvarum* or *S. carlsbergensis*).

In a strict sense the term "brewers yeast" differentiates yeast used in brewing from all other yeasts in that it is a yeast strain which is used to make beer, i.e. a strain of yeast used currently in a beer manufacturing process. Such yeasts must be able to produce a palatable acceptable beer by their fermentative action upon hopped or unhopped malt extract (brewers wort). The primary products of this fermentation are ethanol and carbon dioxide, which are essential constituents of beer. However, not all yeasts belonging to the species *S. cerevisiae* are capable of fulfilling these requirements. Indeed, the critical factor in this respect is believed to be the ability of the yeast strain to form in subtly balanced proportions, quantitatively minor metallic products such as esters, acids, higher alcohols and ketones. A yeast may be unsuitable for brewing because one or more of these minor metabolic products is produced in excessive amounts, either in absolute terms or relative to one another. 'Rainbow, C.A., 1970, in "The Yeasts", eds, Rose, A. H. & Harrison, J. S. Vol. 3, p. 147).

In a general sense brewers yeast is differentiated from other yeasts by the properties which it possesses. Most strains of industrial yeast, unlike laboratory yeast, are incapable of undergoing mating, they are said to be homothallic. Industrial yeasts are usually aneuploid or polyploid, and there is therefore a reduced incidence at which gene mutations are phenotypically detected. Most polyploid strains do not sporulate or they produce spores of very low viability, thus frustrating any meaningful genetic analysis. These factors together tend to confer a measure of phenotypic stability on industrial yeasts which may contribute to their selection for industrial application. Similarly gene dosage which is associated with high ploidy may contribute to the general fitness of such strains for fermentation as compared to haploids and diploids, which generally ferment poorly.

In addition, brewers yeasts have certain technological behaviour which equips them well for interacting with their normal environment, i.e. brewers' hopped wort, for example the ability to ferment at the top of the fermentation vessel (ale yeast) or the bottom of the vessel (lager yeast).

In European Patent Application No. 84308981.4 in the name of Bass PLC, published under No. 147198 there is described and claimed a process for the production of ethanol and a protein of peptide which is heterologous to yeast which comprises fermenting an aqueous carbohydrate-containing medium with a yeast strain which has been genetically modified to be capable of expressing a heterologous protein or peptide, recovering the ethanol so formed, and obtaining the said heterologous protein, or peptide from the fermentation products.

The invention of that Application is based on the discovery that it is possible to use, in an industrial fermentation involving the production of alcohol, genetically modified yeast capable of expressing a heterologous protein or peptide. Surprisingly, it was found that the use of such yeasts is compatible with industiral or brewery fermentation conditions. This means that the excess yeast obtained in the fermentation provides a source of the heterologous protein or peptide and thus has much enhanced industrial value. Further, since the alcohol product remains the principal objective of the fermentation, and the conventional equipment can largely be used with little alteration, the additional cost of producing the higher value yeast product is small, so that the new process may provide an economically viable route to heterologous proteins or peptides which, although valuable, do not command a premium price.

When the process of our aforesaid Application is operated to produce an aqueous potable liquid such as beer, at the end of the fermentation, the fermented liquid is separated from the yeast (and normally any other solid material present in the fermented medium). In these circumstances, it is clearly desirable, and indeed normally essential, that the fermented liquid shall not contain the heterologous protein or peptide, since it is normally unacceptable for the heterologous protein or peptide to be present in a liquid which is to be drunk.

In the said Application it is stated that, in such circumstances, the heterologous protein or peptide may be obtained from the yeast cells.

In the process of the present invention, the fermentation is carried out under conditions such that the yeast reproduces in the usual way but little or no heterologous protein or peptide is produced. Then, normally after the yeast has been separated from the fermented liquid, expression of the heterologous protein or peptide is induced, and the protein or peptide so obtained is separated from the yeast. Operation in this manner avoids the risk that the fermented liquid itself may become contaminated with the heterologous protein or peptide. It also avoids any risk of the alcohol production being compromised by the presence of the heterologous protein or peptide during the fermentation, and in particular by the synthesis of the protein or peptide adversely affecting the ability of the yeast to produce alcohol by fermentation.

According, therefore, to the present invention, a process for the production of ethanol and a protein or peptide which is heterologous to yeast comprises fermenting an aqueous carbohydrate-containing medium with a yeast strain which has been genetically modified to be capable of expressing a heterologous protein or peptide under conditions such that the yeast multiplies but no expression of the said heterologous protein or peptide takes place, recovering the ethanol so formed, inducing expression of the said protien or peptide by the yeast, and obtaining the said heterologous protein or peptide therefrom.

A variety of different systems have been described for expressing heterologous genes in yeast. In most cases these systems depend upon the growth of new cell biomass under conditions which facilitate the efficient transcription and translation of the gene of interest, so as to produce the relevant protein or peptide. However, with some expression systems, manipulation of the contents of the growth medium influences the expression of the heterologous gene. In such cases new cell biomass can be grown without the concomitant production of the heterologous protein or peptide, and gene expression can then be induced by transferring the yeast cells to conditions which favour heterologous gene expression.

It is necessary in the present invention to choose a gene expression system compatible with the contemplated ethanolic fermentation. Some known systems are inappropriate. For example, glycolytic gene promoters of yeast can be used to induce the production of heterologous proteins in yeast cells. Large amount of yeast biomass can be obtained by growth in a medium containing a non-fermentable substrate as a sole carbon source; gene expression can then be induced by the addition of glucose which stimulates the transcription of the glycolytic gene promoter, e.g. PGK (Kingsman and Kingsman, 1982, European Patent Application, 0 073 635, Expression Vectors). A similar system which utilizes the Acid Phosphatase gene (PHO5) promoter can be used for inducing gene expression under conditions of inorganic phosphate starvation (Hinnen *et al*, 1983, European Patent Application, 0 100 561, Yeast hybrid vectors and their use for the production of polypeptides). These known systems for inducing gene expression in yeast require, however, the use of a defined growth medium upon which to produce large amounts of cell biomass. Furthermore, in the case of the glycolytic gene promoter, growth upon a non-fermentable carbon source requires the presence of oxygen. Thus neither system is generally applicable to fermentation by brewing yeast, since the brewing process is substantially or in part anaerobic (oxygen free) and the growth medium is brewers wort, which is a relatively complex mixture of sugars which includes maltose and glucose.

The induction of heterologous gene expression in brewing yeast must be compatible with the demands of the beer fermentation if it is to be used in the present invention. In this respect it is necessary to induce gene expression with an effector or condition which is normally not present in brewers wort and beer. Known gene expression systems for inducing the production of heterologous proteins and polypeptides in yeast and suitable for use in the present invention, include; (i) the heat shock promoter (HSP90) of yeast (Finkelstein, 1983, WO 84/04535, Heat regulated production of selected and fused proteins in yeast) and (ii) the GAL1 promoter of yeast (Botstein *et al*, 1984, UK Patent Application GB 2,137,208A, the use of the GAL1 promoter). These systems utilize heat and galactose, respectively, to stimulate gene expression and each could be applied in the present invention. The application of a galactose regulated system is particularly useful since brewers' wort does not usually contain sufficient galactose to effect the expression of a galactose regulated promoter. Consequently, such galactose-regulated gene expression systems are not functional in brewing yeast during the course of a beer fermentation.

The yeast strain used in the new process must, of course, be suitable for the type of industrial fermentation contemplated. This objective may be secured by carrying out the genetic modification on a yeast strain which is known to have the desired characteristics, since it has been found that the desirable characteristics which make a yeast strain suitable for a particular type of industrial fermentation can be maintained during the genetic modification. For example, where the fermentation is one for producing beer, the yeast strain chosen for genetic modification in preferably a known strain of brewers' yeast currently used in such fermentations. As already noted, such industrial strains of brewers yeast have characteristics different from those of "laboratory yeast", including in particular the ability to ferment hopped brewers wort.

Brewers wort is essentially a hot water extract of malted barley or other grains prepared by steeping and germination and flavoured with hops. The most important parameters with respect to yeast growth and metabolism are carbohydrate and nitrogen (and amino acid) composition. These vary from country to country and brewery to brewery, see, e.g., "Malting and Brewing Science", Vol. 2, Hopped Wort and Beer; by Hough, J. S., Briggs, D. E., Steven R. and Young, T. W., 1982, Chapman and Hall, London and New York, p. 456—498. In general it may be said that brewers wort contains 5 to 10 g of total fermentable sugars per 100 ml of wort, at least half of which is maltose.

Additional factors which influence yeast growth and performance are: (1) *Growth factors*. These include substances such as biotin, thiamine, riboflavin, pyridoxine, pantothenic acid and nicotinic acid. In general brewers wort is a rich source of these factors, which are depleted during yeast growth. (2) *Minerals*. The mineral requirements of brewers yeast resemble those of most living organisms. Brewers wort meets these requirements, supplying trace amounts of metal ions such as iron, potassium, magnesium, zinc, manganese and copper, which are essential nutrients for growth and fermentation.

The most significant difference between a laboratory culture medium and a brewers wort is the sugar composition of the medium. Most laboratory media utilise glucose as the chief source of carbohydrate, whereas maltose is the chief fermentable constituent of wort.

Brewery fermentation normally take the form of anaerobic (oxygen free) fermentations. However, oxygen is a prime requirement for yeast growth in the initial stages of fermentation. Most laboratory fermentations are designed to maximise the yeast biomass yield, whereas beer fermentations concentrate upon ethanol yield and product flavour. Thus the inoculation rate ("pitching rate") at a beer fermentation is higher than would normally be used in the laboratory. Consequently, the number of cell doublings (cell generations) is reduced to between 2 and 4 per fermentation.

The fermentation of beer wort is normally carried out at a temperature within the range of 8 to 25°C, a temperature at the upper end of this range, e.g. 15 to 25°C being used when the product is ale, and a temperature of e.g. 8 to 15°C being used where the product is lager. Under laboratory conditions, yeasts are cultivated at significantly higher temperature, e.g. 25 to 35°C.

Similarly, where the industrial fermentation is one for the production of alcohol which is separated by distillation, it is necessary to use genetically modified yeast obtained from a strain suitable for such fermentation. In such fermentations, the source of carbohydrates may be, for example, grain, potatoes, cassava, sugar cane, sugar beet or a lignocellulosic material and may optionally have been pre-treated, e.g. by chemical, enzymic or physical methods, to convert cellulose and/or starch therein into fermentable sugars.

The genetic modification of yeast may be effected by the application of known techniques. Suitable methods are described in the literature, and particular methods are given in the Examples below.

A wide range of heterologous proteins or peptides may be expressed in the yeast. By way of example mention may be made of enzymes such as beta-lactamase, beta-glucanase, and beta-galactosidase. Other useful heterologous proteins and peptides include materials of human origin and/or useful in therapy, such as human serum albumin and immunoglobulins. Methods are described in the literature for the genetic modification of microorganisms to enable them to express such proteins and peptides.

The following Examples illustrate the invention in more detail. The eight figures of the accompanying drawings show respectively:

1. Construction of plasmid pEHB11
2. Oligonucleotide sequence of the HSA primer
3. DNA sequence of the HSA cDNA gene
4. 5' Non-coding region modification and signal peptide sequence excision
5. Construction of an "authentic" HSA cDNA (MET-HSA)
6. Plasmid pEK113
7. Construction of plasmid pEHB11-MET-HSA
8. Construction of plasmid pET13:1 MET-HSA.

Example 1

Beta-galactosidase production in brewing yeast

Beta-Galactosidase (EC 3.2.1.23) is an enzyme (protein) which hydrolyses the terminal nonreducing beta-D-galactose residues in beta-D-galactosides such as lactose. *E. coli* beta-galactoside is the product of the *Lac Z* gene which is located at map position 8 minutes on the *E. coli* chromosome (Bachmann, B. J., 1983, *Microbiological Reviews*, 47. p. 180). The *Lac Z* gene forms part of the *lac* operon, which has played a central role in the elucidation of the genetic control of gene activity in the prokaryote *E. coli* (Beckwith, J., 1972. In "The Operon" p. 11, eds, Miller, J. H. and Reznikoff, W. S., Cold Spring Harbor Laboratory, Cold Spring Harbor, New York).

In recent years the structural gene for beta-galactosidase (*lac Z*) has been used in DNA manipulations. It has been shown that the amino-terminal end of the beta-galactosidase protein is not essential for enzymatic activity (Muller-Hill, B. and Kania, J., 1974, *Nature, 149,* p. 561). This has facilitated the construction of many gene fusions, in which the 5'-coding segment of the *lac Z* gene (corresponding to the N-terminal end of the protein) has been replaced with other DNA sequences. In these gene fusions, the gene transcriptional promoter of lac Z is replaced by analogous DNA sequences originating from different genes (Casadaban, M. J. *et al*, 1980, *Journal of Bacteriology*, 143, p. 971), but proteins obtained retain beta-galactosidase activity.

The *lac Z* gene of *E. coli* was one of the first prokaryotic chromosomal genes to be expressed in *S. cerevisiae* (Panthier, J. J. *et al*, 1980, *Current Genetics, 2,* p. 109). In this demonstration the native *E. coli Lac Z* gene was sub-cloned into a yeast/*E. coli* shuttle vector (plasmid) and introduced into laboratory strains of yeast which do not possess endogenous beta-galactosidase activity. However, the level of gene expression and thus enzyme production was inefficient using this system. More recently, gene fusions have been constructed in which the transcriptional promoter of the yeast cytochrome Cl gene (*CYCl*) has replaced that of *Lac Z*. These *CYCl-lac Z* fusions have been introduced, by transformation, into laboratory strains of yeast, where they have been shown to produce enzymatically active beta-galactosidase (Guarente, L. and Ptashne, M. 1981, *Proceedings of the National Academy of Sciences, U.S.A, 78,* p. 2199). Detailed analysis of the *CYCl-Lac Z* gene fusions has shown that the levels of beta-galactosidase produced in yeast display the pattern of regulation normally seen for cytochrome c (i.e. a reduction of synthesis in cells grown in glucose). (Guarente, L. and Ptashne, M., 1981). In this way the *Lac Z* gene has been used to study the regulation of gene expression in both prokaryotes and eukaryotes at the level of DNA transcription.

In the yeast CYCl gene, DNA sequences which regulate DNA transcription by RNA polymerase II have been located in two regions upstream (5') of the coding sequences for the cytochrome c protein. One of these regulatory sequences is situated close to where transcription initiates; the other is upstream of the initiation region and contains an activation site which enhances gene expression (UASc) (Guarente, L. and Ptashne, M. 1981; Faye, G. et al, 1981, *Proceedings of the National Academy of Sciences*, U.S.A., *78,* p. 2258). Guarente and co-workers (1982, Proceedings of the National Academy of Sciences, 79, p. 7410) have shown that other genes possess upstream activation sites (UAS), in particular the *GAL10* gene of *S. cerevisiae*. These workers constructed a gene fusion in which the UASc of *CYCl* was replaced by the UAS of the *GAL10* gene. Furthermore, the gene fusion which retains the promoter (transcription initiation region) of *CYCl* is fused to the *Lac Z* gene of *E. coli*, such that an enzymatically active beta-galactosidase is produced in yeast transformed with plasmid DNA carrying the hybrid gene (plasmid pLGSD5).

The regulation of genes involved in galactose metabolism in yeast is well understood. The transcriptional expression of three genes (*GAL1, GAL10* and *GAL7*) encoding enzymes required for the metabolism of galactose is dependent upon a transcriptional activator encoded by the gene *GAL4* (Oshima, Y. 1982, in "The Molecular Biology of the Yeast Saccharomyces: Metabolism and Gene Expression" eds. Broach J. M. *et al*, p. 159. Cold Spring Harbor Laboratory, Cold Spring Harbor, New York). The ability of the *GAL4* activator protein to promote transcription of these genes is reversibly inactivated by the product of the *GAL80* gene, (GAL80 repressor protein). Galactose is thought to bind to the *GAL80* repressor protein thereby freeing the *GAL4* activator protein to promote transcription of the galactose-specific genes. The *GAL10-CYCl* hybrid promoter is subject to induction by galactose in the manner described above (Guarente, L. *et al*, 1982, *Proceedings of the National Academy of Sciences, U.S.A., 79,* p. 7410). Thus beta-galactosidase production is induced by galactose.

The production of E. coli beta-galactosidase in brewing yeast

In order to introduce the beta-galactosidase gene of *E. coli*, expressed from the *GAL10-CYCl* hybrid promoter into brewing yeast strain NCYC240, it was necessary to sub-clone the *CUP-1* gene present on plasmid pET13:1. The accompanying Figure 1 outlines a scheme whereby the *XbaI* DNA fragment of approximately 2.85 kilo base pairs carrying the CUP-1 gene was sub-cloned to the single *XbaI* site of pLGSD5, to form recombinant plasmid pEHB11, containing the GAL10-CYCl promoter.

Brewing yeast strain NCYC240 was prepared for transformation with plasmid pEHB11, by the following procedure. The yeast was grown in 100 ml YEP medium (10 g/litre yeast extract, 20 g/litre peptone) supplemented with 2% w/v glucose to early exponential growth phase; cells were harvested and washed in cold sterile water prior to resuspending in 10 mM Tris HCl buffer at pH 7.6 containing 1M Sorbitol, 10 mM dithiothreitol and 40 µg/ml Symolyase 6000 (Kirin Brewery Co. Ltd) at 30°C. After 1.5 hours incubation the yeast cells are converted into spheroplasts which are harvested by centrifugation and washed three times in 20 ml of a solution containing 1M Sorbitol, 10

mM $CaCl_2$, 10 mM Tris HCl at pH 7.6. Spheroplasts are finally resuspended in 1 ml of a solution of 1M Sorbitol, 10 mM $CaCl_2$, 10 mM Tris HCl at pH 7.6 and 100 µl is added to 15 µl of plasmid pEHB11. This mixture is incubated at room temperature for 30 minutes prior to the addition of 1 ml of a solution of 40% polyethylene glycol 4000, 10 mM $CaCl_2$, 10 mM Tris HCl at pH 7.6. After 2—5 minutes spheroplasts are harvested by centrifugation and gently resuspended in 0.5 ml NEP Sorbitol medium ($MgSO_4 \cdot 7H_2O$ 2 g/l, $(NH_4)_2 SO_4$ 2 g/l, $KH_2PO_4$ 3 g/l, $CaCl_2 \cdot 2H_2O$ 0.25 g/l, yeast extract 2 g/l, peptone 3 g/l glucose 40 g/l, 1M sorbitol) and incubated at 28°C for 1 hour before plating in molten NEP Sorbitol supplemented with 3% w/v agar and 0.2 mM $CuSO_4 . 7H_2O$. Plates were incubated for 4—6 days at 28°C after which time copper resistant colonies were picked and transferred to NEP supplemented with 2% agar and 0.2 mM $CuSO_4 . 7H_2O$. Transformants of NCYC240 harbouring the plasmid pEHB11 were verified by their ability to grow on NEP agar containing 1 mM $CuSO_4 . 7H_2O$. In addition, these transformants could be detected by growth on M63 medium containing galactose and the chromogenic indicator Xgal (5-bromo-4-chloro-3-indolyl-beta-D-galactoside) according to the procedure of Guarente (1983), in "Methods in Enzymology, Recombinant DNA, Vol. 107, eds. Wu, R. and Grossman, L., p. 181). Yeast colonies producing beta-galactosidase (harbouring pEHB11) develop a blue/green colour which is not apparent in those colonies lacking pEHB11. Thus it can be shown by means of a simple plate assay that brewing yeast transformed with the plasmid pEHB11 is capable of producing enzymatically active beta-galactosidase.

The yeast strain NCYC 240 (pEHB11) has been deposited at the National Collection of Yeast Cultures, Colney Lane, Norwich NR4 7AU, United Kingdom on December 12th, 1984 under No. NCYC 1547.

Induction of beta-galactosidase production in brewing yeast

Brewing yeast strains NCYC240 (pEHB11) and NCYC240 (pET13:1) described in the aforesaid Patent Application were grown in aerobic shake flask culture (NEP medium plus 2% glucose and 0.2 mM $CuSO_4 \cdot 7H_2O$) at 28°C. Late stationary phase cells were harvested from the culture medium and assayed for beta-galactosidase activity as described by Miller (In "Experiments in Molacular Genetics" ed. Miller, J. H., Cold Spring Harbor Laboratory, Cold Spring Harbor, New York, 1972).

Cells were washed×2 in distilled water and resuspended in Z buffer (16.1 g/l $Na_2HPO_4 \cdot 7H_2O$, 5.5 g/l $NaH_2PO_4 \cdot H_2O$, 0.75 g/l KCl, 0.246 g/l $MgSO_4 \cdot 7H_2O$ 2.7 ml/l 2-mercaptoethanol). The absorbance at 600 nm was determined and the cells were permeabilized by the addition of 3 drops of chloroform, 2 drops of 0.1% sodium dodecyl sulphate (SDS) and vortexing for 10 sec. To 1 ml of permeabilized cells in Z buffer was added 0.2 ml of O-nitrophenol-galactoside (ONPG) (4 mg/ml in phosphate buffer, pH 7) and the reaction mixture was incubated at 28°C. The reaction was stopped by the addition of 0.5 ml 1M $Na_2CO_3$, cell debris removed, and the absorbance at 420 nm determined. Beta-Galactosidase units were calculated as described by Miller (1972).

Assays revealed that NCYC 240 (pET13:1) did not possess any endogenous beta-galactosidase activity, whereas NCYC 240 (pEHB11) produced approximately 1.05 units of beta-galactosidase. When cells of NCYC 240 (pEHB11) were resuspended in 2 volumes of 2% galactose solution and incubated at 28°C for a prolonged period of time, beta-galactosidase activity was induced to a considerably higher level. Thus, after five hours induction, 135 units of beta-galactosidase were obtained, without any increase in cell number, whereas after 23 hours induction and a 2 fold increase in cell number 4154 units of beta-galactosidase were obtained. When either strain NCYC 240 or NCYC 240 (pET13:1) were subjected to the same treatment there was no detectable beta-galactosidase.

Galactose induction of beta-galactosidase production in brewing yeast following a beer fermentation

Plasmid pEHB11 are transformed, as described previously, into a proprietary strain of lager yeast, BB10.1, BB10.1 (pEHB11) and NCYC 240 (pEHB11) were grown in NEP medium plus 2% glucose and 0.2 mM $CuSO_4 . 7H_2O$ at 28°C, yeast were harvested and pitched (inoculated) into air saturated brewers wort. Fermentation proceeded anaerobically at 12°C until there was no further reduction in the specific gravity of the beer. Fermentation profiles were closely monitored and direct comparisons were made with fermentations performed on the same wort with the parental brewing yeast. NCYC 240 and BB10.1 which had not been transformed with plasmid pEHB11. In all cases the genetically modified yesat fermented the wort at the same rate and to the same degree of attenuation as the parental yeast strains. BB10.1 (pEHB11) and NCYC 240 (pEHB11) were harvested from their respective beers by centrifugation and following two washes in water were assayed for beta-galactosidase activity. Neither yeast strain produced in excess of 1.0 units of beta-galactosidase when assayed in this manner. However, following resuspension in 2% w/v galactose significant quantities of beta-galactosidase were obtained. Optimal conditions for galactose induction of beta-galactosidase activity were defined as follows: (i) 2—3% w/v galactose, (ii) minimal salts medium (1.7 g/l yeast nitrogen base without amino acids and ammonium sulphate, 5 g/l ammonium sulphate, 2.5% w/v casamino acids), (iii) cell density of 0.1 to 30%, preferably 10%, w/v (wet yeast pellet/induction medium) (iv) presence of molecular oxygen. Employment of these conditions resulted in the routine production of 2000—1200 units of beta-galactosidase in NCYC 240 (pEHB11) and BB10.1 (pEHB11) following 24 hours residence in induction medium.

Strain NCYC 240 (pEHB11) was further evaluated under beer production conditions. In this case the yeast was used to ferment an all malt ale wort in a five-barrel (5X 163.7 litres) experimental brewery. The beer produced was conditioned, fined and packaged into bottle, after which it was compared with a control beer fermented by the parental yeast NCYC 240, produced under identical conditions. Qualitative organoleptic analysis did not reveal any significant difference between the two products. Thus it is apparent that brewing yeast can be successfully genetically modified such that they are capable of producing significant quantities of heterologous protein following a post-fermentation induction, without adversely influencing the ability of the yeast to produce the primary beer product.

Example II

Production of human serum albumin protein in brewing yeast

The cloning of the human Serum Albumin (HSA) cDNA was carried out using a specific oligonucleotide primer (see Baralle, F. E., 1977, *Cell, 10*, 549—558; Noyes, B. E. *etal* 1979, *Proceedings of the National Academy of Science, USA, 76*, 1770—1774; Hudson, P. *et al*, 1981, *Nature, 291*, 127—131). By selecting a favourable region of the known amino acid sequence of the HSA protein (Dayhoff, M. O., 1976 Atlas of Protein Sequences and Structures Nat. Biomed, Res. Foundation Washington) it was possible to predict from the genetic code eight 14 long oligonucleotides, one of which should be exactly complementary to the HSA messenger RNA (mRNA) (see Figure 2 of the accompanying drawings). The oligonucleotides were synthesized simultaneously (by the method of Wallace, R. B., (1981, *Nucleic Acids Research, 9*, 879—894) using the solid phase phosphotriester method developed by Gait, M. K., *et al*, (1980, *Nucleic Acids Research, 8*, 1081—1096). This mixture of synthetic oligonucleotides was used as a primer for cDNA synthesis using human liver RNA as a template (see Baralle, F.E., 1977, *Cell, 12*, 1085—1095). The syntehsized cDNA was fractionated on a denaturing gel and an appropriately sized band was eluted. This single stranded cDNA was then converted to double strand by the "loop back" reaction with Klenow DNA polymerase (see Wickens, M. P., *et al*, 1978 *Journal of Biological Chemistry, 253*, 2483—2495). The resulting double stranded cDNA was digested with *TaqI* and ligated to M13mp9 vector which had been *AccI*-restricted and alkaline phosphatase-treated and the ligation mixture was transformed into the *E. coli* strain JM101 (Messing, J. and Vieira, J., 1981, Analects, 9, (8), 1). The DNA sequence analysis (determined by the method of Sanger, F. *et al.* 1980, *Journal of Molecular Biology, 143*, 161—178) of a sample of the recombinant phage allowed the identification of two clones herein designated M13ALB1 and M13ALB2 (Fig. 3). These clones included the DNA sequence of the HSA cDNA between the nucleotides 144—692 (M13ALB1) and 692—955 (M13ALB2) (Fig. 3).

A cDNA library was prepared synthesizing cDNA with total liver mRNA as template and using oligo thymine dT12—18 as primer. The cDNA's were converted to double strands as described previously and the single strand hairpin loop structure was digested with S1 nuclease (Efstratiadis, A. *et al*, 1976, *Cell, 7*, 279—288). The products were purified on a Sephacryl S 300 column and the eventual 5′ overhanging ends of the cDNA were repaired by "filling in" with the Klenow fragment of DNA polymerase I. The cDNA molecules at this stage were predominantly blunt ended so they were ligated to the *PvuII* site of pAT153*PvuII*8 (previously treated with alkaline phosphatase to avoid its circulation) (Anson, D. et. al. The EMBO Journal, *3*, 1984, pp 1053—1064) and transformed into *E. coli* strain MC1061 (Casadaban, M. T., & Cohen, S. N., 1980 *Journal of Molecular Biology, 138*, 179—207) or another appropriate strian. Approximately 10,000 recombinant colonies were produced from 10 µg of mRNA. The cDNA in these colonies was screened for HSA DNA sequences using radioactive probes prepared from M13ALB1 and M1ALB2 by nick translation (Rigby, P.W.J. *et al*, 1977, *Journal of Molecular Biology, 113*, 237—251). Colonies of *E. coli* strain MC1061 harbouring recombinant cDNA clones possessing DNA sequence homology with the HSA gene were isolated as described by Baralle, F. E. *et al* (1980, Nucleic *Acids Research, 8*, 4393—4404). Full length cDNA clones were characterized by DNA sequence analysis (Maxam, A. M. and Gilbert, W., 1980, *Methods in Enzyology, 65*, 499—560; Sanger, F. *et al*, 1980, *Journal of Molecular Biology, 143*, 161—178) and were found to include the entire HSA cDNA gene (Fig. 3). The 5′ non-coding region and the signal peptide sequence of the full length HSA cDNA were excised in the way shown in Figure 4 to produce plasmid pAT135ALB.

Construction of a MET-HSA cDNA

The strategy for the construction of a MET-HSA cDNA employed generally known techniques: a summary is shown in Figure 5. The 1843 base pair BamHI fragment from pAT153 ALB was isolated, and made flush ended by nuclease SI treatment (Maniatis *et al.* 1982, *Molecular Cloning: A Laboratory Manual, Cold Spring Harbor*, USA) at the *XBaI* site of M13mp19 (Messing, 1983, *Methods in Enzymology, Recombinant, 101*, 20—78), the latter having been similarly made flush ended by nuclease S1 treatment. Following transfaction of *E. coli* JM101, lysates were prepared from several plaques and single stranded (SS) DNA isolated from the mature bacteriophage particles. Subsequent to DNA sequence analysis (Sanger, 1977 *et al. Proc. Nat. Acad. Sci., USA, 74*, 5463) the recombinant M13mp19.7 was identified. Double stranded (RF) DNA was prepared and cleaved at the newly created unique *XhoI* site by

treatment with the latter restriction endonuclease, followed by S1 nuclease digestion to remove the 5′ single strand extensions.

These manipulations served to expose the first codon (GAT) for the mature, native polypeptide sequence. Next, a synthetic oligonucleotide whose 5′ ends were unphosphorylated was inserted at the modified *XhoI* site. As previously, recombinants were analysed directly by DNA sequence determination following transfection and SS NDA preparation. The sequence of the relevant portions of the such recombinant, M13 mp 19.7 met/9, is shown (Figure 5). This deviates from the expected sequence, also shown in Figure 5; this deviation is most probably due to secondary structure formation about the double *Bam*HI site and subsequent excition of the non-bonded regions of the intrastrand partial duplex. These deviations are inconsequential since the sole purpose for the linker insertion was to introduce an initiation codon (ATG) and a *Bam*HI site immediately upstream from the HSA coding sequence, which was achieved. For convenience a further *Bam*HI site was introduced 3′ to the MET-HSA coding sequence in M13mp19.7 met/9. In this instance RF DNA of the latter molecule was cleaved at the unique *SalI* site, originating in the cloning linker of M13mp19, and this species made flush ended by treatment with *E. coli* DNA polymerase I (the large fragment or "Klenow" fragment (Maniatis *et al*, 1982, *Molecular Cloning: A Laboratory Manual*, Cold Spring Harbor, USA)). The same linker used above was inserted at this modified site. Following RF DNA preparation and *Bam*HI digestion a suitable recombinant clone was identified and this was designated M13mp19.7 met/9.1. A single *Bam*HI fragment carrying the full length MET-HSA cDNA gene was subsequently cloned in the single *Bam*HI site of the *E. coli* vector pBR322 (Bolivar *et al*, 1977, *Gene*, *2*, 95—113) to derive a plasmid designated pEK113 (Figure 6). Plasmid pEK113 was transformed into the *E. coli* strain MC1061 (Casadaban & Cohen, 1980, Journal of Molecular Biology, *138*, 179—207) which was deposited in the National Collection of Industrial Bacteria (NCIB) Torry Research Station, PO Box 31, 135, Abbey Road, Aberdeen, Scotland, AB9 8DG on April 3rd 1986 and has the accession number NCIB 12242.

The inducible expression of HSA-like protein in brewing yeast

In order to produce HSA-like protein in brewing yeast by the inducible gene expression system it is necessary to fuse the MET-HSA cDNA coding sequence in an appropriate expression signal. The *GAL10-CYCI* hybrid promoter which controls the galactose inducible expression of the *E. coli lacZ* gene (β-galactosidase production) has general application as a system for mediating the galactose inducible expression of heterologous genes in brewing yeast. To take advantage of this, the MET-HSA cDNA in fused to the *GAL10-CYCI* promoter present in plasmid pEHB11. Plasmid pEHB11 is digested with the restriction endonuclease *Bam*HI and the *Bam*HI fragment carrying the MET-HSA cDNA from pEK113 is inserted. The orientation of insertion is determined by characterising the recombinant plasmids generated with restriction endonucleases and separating the fragments thus formed by agarose gel electrophoresis. A recombinant plasmid is isolated in which the 5′ MET-HSA cDNA has fused with the 3′ *GAL10-CYCI* hybrid promoter, such that following DNA sequence analysis (Maxam, A. M. and Gilbert, W., 1980, Methods in Enzymology, *65*, 499—560) the junction of the fusion has the DNA sequence 5′...TTAATA *ATG* ACC GGA TCC ATG GAT...3′ (See Figure 7). This plasmid is designated pEHB11-MET-HSA and may be introduced into brewing yeast strains NCYC 240 and BB10.1 by transformation and selection for copper resistance as described previously. Brewing yeast strains transformed with plasmid pEHB11-MET-HSA are expected in produce under appropriate conditions an HSA-like protein consisting of five additional amino acids prior to the first N terminal amino acid of mature HSA (N-methionine, threonine, glycine, serine, methionine-HSA-C).

Strain NCYC 240 and BB10.1 harbouring pEHB11-MET-HSA may be grown to stationary phase in NEP glucose medium supplemented with 0.2 mM $CuSO_4 . 7H_2O$. Cells are then harvested by centrifugation and washed in water before resuspending for 24 hours under optimal conditions for galactose induction of gene expression, described previously. Crude cell free extracts of induced (plus galactose) and uninduced (without galactose) cultures are prepared by disrupting the cells on a Braun homogeniser in 0.1M sodium phosphate pH 7.5, 1 mM phenylmethylsulfonyl fluoride and 5 mM 2-mercaptoethanol). Glass beads and cell debris are removed by centrifugation (2000×g for 2 minutes) and the supernatant is clarified by recentrifugation (8000×g for 10 minutes). The resultant cell extracts are assayed for the presence of Human Serum Albumin protein by SDS: polyacrylamide gel electrophoresis followed by Western blotting (see Mellor, J. *et al*, 1985, *Gene, 33,* p215). The results of this assay indicate that those cultures which had been induced in the presence of galactose contained significant quantities of intracellular human Serum Albumin-like protein, whereas uninduced cultures do not.

A similar galactose induction of Human Serum Albumin-like protein production can be obtained with both NCYC 240 (pEHB11-MET-HSA) and BB10.1 (pEHB11-MET-HSA) harvested from a beer fermentation in which the yeast strain has first been used to ferment brewers wort under anaerobic fermentative conditions. Yeast harvested at the end of the beer fermentation does not contain detectable qunatities of Human Serum Albumin-like protein, whereas following 24 hours of galactose induction significant amounts of protein can be detected. It is further noted that brewing yeasts genetically modified in the manner described are capable of fermenting brewers wort at the same rate and to the same degree of attenuation as their unmodified parental counterparts; beers thus produced are indistinguishable from controls on organoleptic analysis.

The inducible expression of MET-HSA in brewing yeast

The *Gal10-CYCI-HSA* fusion plasmid pEHB11-MET-HSA can be shown to produce an HSA-like protein in brewing yeast following post-fermentation induction in galactose induction medium. This protein is described as HSA-like since it possesses five extra amino acids at the N-terminus, as described previously. In order to produce a more "authentic" HSA protein it is necessary to construct a *GAL10-CYCI* hybrid promoter transcriptional fusion with the MET-HSA cDNA in which the 5' translational ATG codon (methionine) is supplied by the MET-HSA cDNA. For this purpose the ATG$^-$ *GAL10-CYCI* promoter carried by the publicly available plasmid 62 can be used (Guarente, 1983, *Methods in Enzymology, Recombinant DNA, 101*, 181—191). Plasmid G2 is digested with the restriction endonuclease *Bam*HI and ligated with ~1.8 kilo base-pair MET-HSA cDNA *Bam*HI fragment from pEK113. The orientation of insertion is confirmed by restriction endonuclease digestion and shows that the 5' MET-HSA cDNA is fused to the 3' *GAL10-CYCI* ATG$^-$ promoter. This plasmid is designated G2 MET-HSA (see Figure 8). Plasmid G2 MET-HSA carries an *E. coli* plasmid origin of DNA replication, the B-lactamase gene conferring ampicillin resistance in *E. coli*, the 2 μm origin of DNA replication of yeast, the *URA-3* gene of yeast (facilitating selection by complementation of *ura-3* auxotrophs in laboratory yeast) and the *GAL10-CYCI*-MET-HSA fusion cassette. Transcription of the MET-HSA gene is expected to result in the synthesis of a messenger RNA which will initiate translation at the first ATG in the message, and this ATG is provided by the MET-HSA gene.

In order to regulate the expression of the MET-HSA gene in brewing yeast, and thus the production of an "authentic" recombinant Human Serum Albumin protein, it is necessary to fuse the *GAL10-CYCI*-MET-HSA expression unit to the *CUP-1* gene. This is accomplished by digesting plasmid G2-MET-HSA with the restriction endonuclease *Hind*III, which cleaves upstream (5') of the *URA-3* gene and downstream (3') of the MET-HSA coding sequence (see Figure 8), and sub-cloning this fragment into the *Hind*III site of pET13:1 to form plasmid designated pET13:1-MET-HSA (Figure 8).

Plasmid pET13:1-MET-HSA is subsequently transformed into the brewing yeast strains NCYC 240 and BB10.1 by the methods described previously. Brewing yeast strains harbouring plasmid pET13:1-MET-HSA are gorwn to stationary phase in NEP glucose medium supplemented with 0.2 m. $CuSO_4 . 7H_2O$. Cells are harvested and processed prior to the galactose induction of gene expression, as described previously. Cell extracts were prepared and assayed for the presence of Human Serum Albumin protein, as described previously. In all cases brewing yeast strains harbouring the *GAL10-CYCI*-MET-HSA (pET13:1-MET-HSA) produced significant quantities of Human Serum Albumin protein. When the same strains are grown on brewers wort under anaerobic conditions, followed by induction of gene expression and HSA assay as described previously, significant quantities of Human Serum Albumin can be detected. The yeast harvested at the end of a beer fermentation does not possess any detectable Human Serum Albumin protein prior to galactose induction.

**Claims**

1. Process for the production of ethanol and a protein or peptide which is heterologous to yeast which comprises fermenting an aqueous carbodrate-containing medium with a yeast strain which has been genetically modified to be capable of expressing a heterologous protein or peptide under conditions such that the yeast multiplies but no expression of the said heterologous protein or peptide takes place, recovering the ethanol so formed, inducing expression of the said protein or peptide by the yeast, and obtaining the said heterologous protein or peptide therefrom.

2. Process according to claim 1 in which the said yeast contains a plasmid having the GAL10-CYCI hybrid promoter and expression of the said protein or peptide is induced by exposing the said multiplied yeast to galactose.

3. Process according to claim 1 or 2 in which the said protein or peptide is human serum albumin or a human serum albumin-like protein.

4. Process according to any of claims 1 to 3 in which the ethanol is recovered in the form of an aqueous potable liquid which is substantially free from yeast and which contains substantially all the water and ethanol of the said fermented medium.

5. Process according to any of claims 1 to 3 in which the ethanol is recovered from the said fermented medium in the form of an ethanolic distillate.

6. Process according to claim 4 in which the aqueous carbohydrate-containing medium contains maltose as the major sugar present.

7. Process according to claim 6 in which the aqueous carbohydrate-containing medium is a barley malt-based beer wort.

8. Process according to claim 4, 6 or 7 in which the fermentation is effected at 8 to 25°C.

9. Process according to claim 5 in which the aqueous carbohydrate-containing medium is a fermentation medium for the production of potable distilled ethanol or power ethanol.

10. Process according to claim 9 in which the said medium is based on grain, potatoes, cassava, sugar cane, sugar beet or a lignocellulosic material optionally pretreated to convert cellulose and/or starch therein into fermentable sugars.

11. Process according to any one of claims 1 to 10 in which the fermentation is a substantially anaerobic fermentation.

12. Process according to any one of claims 1 to 11 in which the yeast used is a genetically engineered modification of an industrial strain of *Saccharromyces cerevisiae*, or *S. carlsbergensis*.

**Patentansprüche**

1. Verfahren zur Herstellung von Ethanol und eines zu Hefe heterologen Proteins oder Peptids durch Fermentieren eines wäßrigen, kohlenhydrathaltigen Mediums mit einem Hefestamm, der derart genetisch modifiziert wurde, daß er zur Expression eines heterologen Proteins oder Peptids fähig ist, unter Bedingungen, unter denen zwar eine Vermehrung der Hefe, jedoch keine Expression des heterologen Proteins oder Peptids stattfindet, Gewinnung des gebildeten Ethanols, Anslösung der Expression des Proteins oder Peptids durch die Hefe und Gewinnung des heterologen Proteins oder Peptids daraus.

2. Verfahren nach Anspruch 1, dadurch gekennzeichnet, daß die Hefe ein Plasmid mit dem GAL 10-CYCI-Hybridpromotor enthält und die Expression des Proteins oder Peptids ausgelöst wird, indem man die vermehrte Hefe einer Galactoseeinwirkung aussetzt.

3. Verfahren nach Anspruch 1 oder 2, dadurch gekennzeichnet, daß es sich bei dem Protein oder Peptid um Humanserumalbumin oder ein humanserumalbuminartiges Protein handelt.

4. Verfahren nach einem der Ansrpüche 1 bis 3, dadurch gekennzeichnet, daß das Ethanol in Form einer praktisch hefefreien und praktisch das gesamte Wasser und das gesamte Ethanol des fermentierten Mediums enthaltenden wäßrigen trinkbaren Flüssigkeit gewonnen wird.

5. Verfahren nach einem der Ansrpüche 1 bis 3, dadurch gekennzeichnet, daß das Ethanol aus dem fermentierten Medium in Form eines Ethanoldestillats gewonnen wird.

6. Verfahren nach Anspruch 4, dadurch gekennzeichnet, daß das wäßrige, kohlenhydrathaltige Medium Maltose als hauptsächlich vorhandenen Zucker enthält.

7. Verfahren nach Anspruch 6, dadurch gekennzeichnet, daß es sich bei dem wäßrigen, kohlenhydrathaltigen Medium um eine Bierwürze auf Gerstenmalzbasis handelt.

9. Verfahren nach Anspruch 4, 6 oder 7, dadurch gekennzeichnet, daß die Fermentierung bei 8° bis 25°C durchgeführt wird.

9. Verfahren nach Anspruch 5, dadurch gekennzeichnet, daß es sich bei dem wäßrigen, kohlenhydrathaltigen Medium um ein Fermentationsmedium für die Gewinnung von destilliertem Trinkethanol oder Antriebsethanol handelt.

10. Verfahren nach Anspruch 9, dadurch gekennzeichnet, daß das Medium auf Korn, Kartoffeln, Maniok, Zuckerrohr, Zuckerrüben oder einem gegebenenfalls zur Umwandlung von darin enthaltender Cellulose und/oder Stärke zu vorgärbaren Zuckern vorbehandelten Lignocellulosematerial basiert.

11. Verfahren nach einem der Ansrpüche 1 bis 10, dadurch gekennzeichnet, daß die Fermentation im wesentlichen anaerob geführt wird.

12. Verfahren nach einem der Ansprüche 1 bis 11, dadurch gekennzeicnet, daß es sich bei der verwendeten Hefe um einen gentechnologisch modifizierten Industriestamm von Saccharromyces cerevisiae oder S. carlsbergensis handelt.

**Revendications**

1. Procédé pour la production d'éthanol et d'une protéine ou d'un peptide qui sont hétérologues de la levure, comprenant la fermentation d'un milieu aqueux contenant des carbohydrates avec une souche de levure ayant été modifiée génétiquement pour être capable de dégager une protéine ou un peptide hétérologues dans des conditions telles que la levure se multiplie mais qu'il ne se produise pas de dégagement de ladite protéine ou dudit peptide hétérologues, en récupérant l'éthanol ainsi formé, en provoquant le dégagement de ladite protéine ou dudit peptide par la levure, et en obtenant ladite protéine ou ledit peptide hétérologues à partir de ce dégagement.

2. Procédé selon la revendication 1 dans lequel ladite levure contient un plasmide ayant le promoteur hybride GAL 10-CYCI, et le dégagement de ladite protéine ou dudit peptide est induit en exposant ladite levure multipliée au galactose.

3. Procédé selon la revendication 1 ou 2, dans lequel ladite protéine ou ledit peptide est l'albumine du sérum humain ou une protéine analogue à l'albumine du sérum humain.

4. Procédé selon l'une quelconque des revendications 1 à 3, dans lequel l'éthanol est récupéré sous la forme d'un liquide aqueux potable qui est pratiquement exempt de levure et qui contient pratiquement toute l'eau et tout l'éthanol dudit milieu fermenté.

5. Procédé selon l'une quelconque des revendications 1 à 3, dans lequel l'éthanol est récupéré à partir dudit milieu fermenté sous la forme d'un distillat éthanolique.

6. Procédé selon la revendication 4, dans lequel le milieu aqueux contenant des carbohydrates contient du maltose en tant que principal sucre présent.

7. Procédé selon la revendication 6, dans lequel le milieu aqueux contenant des carbohydrates est un moût de bière d'orge à base de malt.

8. Procédé selon la revendication 4, 6 ou 7, dans lequel la fermentation est effectuée à une température de 8 à 25°C.

9. Procédé selon la revendication 5, dans lequel le milieu aqueux contenant des carbohydrates est un milieu de fermentation pour la production d'éthanol distillé buvable ou d'éthanol carburant.

10. Procédé selon la revendication 9, dans lequel ledit milieu est à base de grains, de pommes de terre, de manioc, de canne à sucre, de betterave sucrière ou d'un matériau lignocellulosique optionnellement pré-traité pour convertir la cellulose et/ou l'amidon contenus en sucres fermentescibles.

11. Procédé selon l'une quelconque des revendications 1 à 10, dans lequel la fermentation est pratiquement anaérobe.

12. Procédé selon l'une quelconque des revendications 1 à 11, dans lequel la levure utilisée est une modification, obtenue par génie génétique, d'une souche industrielle de *Saccharomyces cerevisiae* ou de *S. carlsbergensis*.

*Fig.1.* Construction of plasmid pEHB11

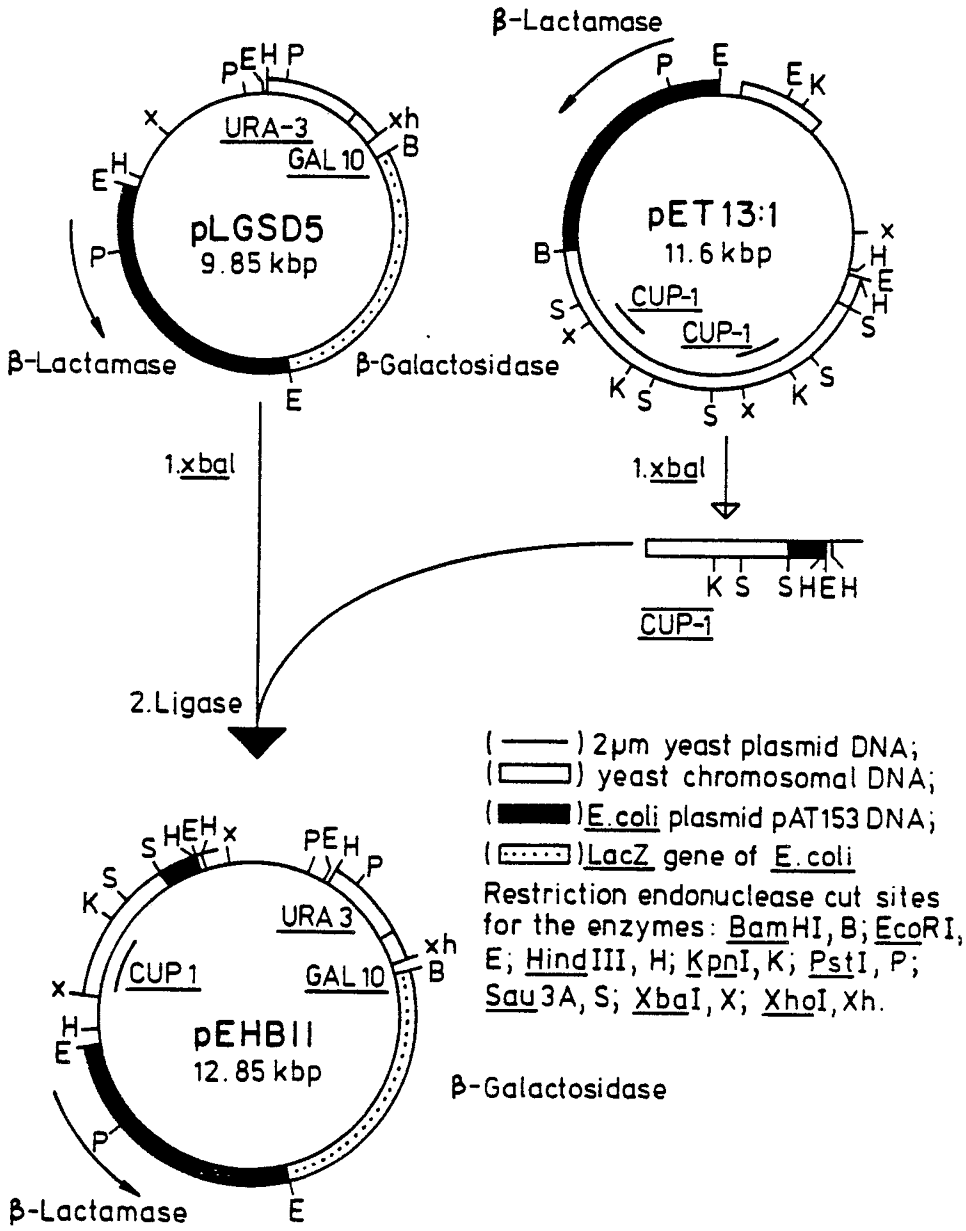

*Fig. 2.* Oligonucleotide sequence of the HSA primer

| | | 295 | 296 | 297 | 298 | 299 | |
|---|---|---|---|---|---|---|---|
| HSA | | 295 | 296 | 297 | 298 | 299 | |
| Amino acid sequence: | | Asn | Asp | Glu | Met | Pro | |
| mRNA: | 5' | AAY | GAY | GAR | AUG | CC N | 3' |
| + Predicted Primer: | 3' | TTR | CTR | CTY | TAC | GG I | 5' |
| * 14 long oligonucleotide primer: used for HSA | | TTA G | CTA G | CTT C | TAC | GG | |

+ From the genetic code an mRNA sequence can be predicted with 3 ambiguities [R indicates a purine (adenine or guanine), Y indicates a pyrimidine (thymine or cytosine) ]

* The primer sequence was designed to be complementary to the MRNA region coding for amino acids 295-298. Hence a mixture of the 8 possible complementary oligonucleotides was simultaneously synthesized.

*Fig. 3.* DNA Sequence of the Human serum Albumin c DNA gene

(Pre-pro)
M K W V T F I S L L F L F S S
AGGATGTCTTCTGGCAATTTCATATAAGTATTTTTTCAAAAATGTCTCTTCTGTCAACCCCACGCCTTTGGCACAATGAAGTGGGTAACCTTTATTTCCCTTCTTTTTCTCTTTAGCTCG
10 20 30 40 50 60 70 80 90 100 110 120

MI3ALB1

(Mature)
A Y S R G V F R R D A H K S E V A H R F K D L G E E N F K A L V L I A F A Q Y L
GCTTATTCCAGGGGTGTGTTTCGTCGAGATGCACACAAGAGTGAGGTTGCTCATCGGTTTAAAGATTTGGGAGAAGAAAATTTCAAAGCCTTGGTGTTGATTGCCTTTGCTCAGTATCTT
130 140 150 160 170 180 190 200 210 220 230 240

Taq I (144)
Q Q C P F E D H V K L V N E V T E F A K T C V A D E S A E N C D K S L H T L F G
CAGCAGTGTCCATTTGAAGATCATGTAAAATTAGTGAATGAAGTAACTGAATTTGCAAAAACATGTGTAGCTGATGAGTCAGCTGAAAATTGTGACAAATCACTTCATACCCTTTTTGGA
250 260 270 280 290 300 310 320 330 340 350 360

D K L C T V A T L R E T Y G E M A D C C A K Q E P E R N E C F L Q H K D D N P N
GACAAATTATGCACAGTTGCAACTCTTCGTGAAACCTATGGTGAAATGGCTGACTGCTGTGCAAAACAAGAACCTGAGAGAAATGAATGCTTCTTGCAACACAAAGATGACAACCCAAAC
370 380 390 400 410 420 430 440 450 460 470 480

L P R L V R P E V D V M C T A F H D N E E T F L K K Y L Y E I A R R H P Y F Y A
CTCCCCCGATTGGTCAGACCAGAGGTTGATGTGATGTGCACTGCTTTTCATGACAATGAAGAGACATTTTTGAAAAAATACTTATATGAAATTGCCAGAAGACATCCTTACTTTTATGCC
490 500 510 520 530 540 550 560 570 580 590 600

P E L L F F A K R Y K A A F T E C C Q A A D K A A C L L P K L D E L R D E G K A
CCGGAACTCCTTTTCTTTGCTAAAAGGTATAAAGCTGCTTTTACAGAATGTTGCCAAGCTGCTGATAAAGCTGCCTGCCTGTTGCCAAAGCTCGATGAACTTCGGGATGAAGGGAAGGCT
610 620 630 640 650 660 670 680 690 700 710 720

MI3ALB2

Taq I (692)
S S A K Q R L K C A S L Q K F G E R A F K A W A V A R L S Q R F P K A E F A E V
TCGTCTGCCAAACAGAGACTCAAATGTGCCAGTCTCCAAAAATTTGGAGAAAGAGCTTTCAAAGCATGGGCAGTGGCTCGCCTGAGCCAGAGATTTCCCAAAGCTGAGTTTGCAGAAGTT
730 740 750 760 770 780 790 800 810 820 830 840

S K L V T D L T K V H T E C C H G D L L E C A D D R A D L A K Y I C E N Q D S I
TCCAAGTTAGTGACAGATCTTACCAAAGTCCACACGGAATGCTGCCATGGAGATCTGCTTGAATGTGCTGATGACAGGGCGGACCTTGCCAAGTATATCTGTGAAAATCAGGATTCGATC
850 860 870 880 890 900 910 920 930 940 950 960

Taq I (955)
S S K L K E C C E K P L L E K S H C I A E V E N D E M P A D L P S L A A D F V E
TCCAGTAAACTGAAGGAATGCTGTGAAAAACCTCTGTTGGAAAAATCCCACTGCATTGCCGAAGTGGAAAATGATGAGATGCCTGCTGACTTGCCTTCATTAGCTGCTGATTTTGTTGAA
970 980 990 1000 1010 1020 1030 1040 1050 1060 1070 1080

S K D V C K N Y A E A K D V F L G M F L Y E Y A R R H P D Y S V V L L L R L A K
AGTAAGGATGTTTGCAAAAACTATGCTGAGGCAAAGGATGTCTTCCTGGGCATGTTTTTGTATGAATATGCAAGAAGGCATCCTGATTACTCTGTCGTGCTGCTGCTGAGACTTGCCAAG
1090 1100 1110 1120 1130 1140 1150 1160 1170 1180 1190 1200

## Fig.3.(Contd.)

```
T  Y  E  T  T  L  E  K  C  C  A  A  A  D  P  H  E  C  Y  A  K  V  F  D  E  F  K  P  L  V  E  E  P  Q  N  L  I  K  Q  N
ACATATGAAACCACTCTAGAGAAGTGCTGTGCCGCTGCAGATCCTCATGAATGCTATGCCAAAGTGTTCGATGAATTTAAACCTCTTGTGGAAGAGCCTCAGAATTTAATCAAACAAAAC
      1210      1220      1230      1240      1250      1260      1270      1280      1290      1300      1310      1320

C  E  L  F  K  Q  L  G  E  Y  K  F  Q  N  A  L  L  V  R  Y  T  K  K  V  P  Q  V  S  T  P  T  L  V  E  V  S  R  N  L  G
TGTGAGCTTTTTAAGCAGCTTGGAGAGTACAAATTCCAGAATGCGCTATTAGTTCGTTACACCAAGAAAGTACCCCAAGTGTCAACTCCAACTCTTGTAGAGGTCTCAAGAAACCTAGGA
      1330      1340      1350      1360      1370      1380      1390      1400      1410      1420      1430      1440

K  V  G  S  K  C  C  K  H  P  E  A  K  R  M  P  C  A  E  D  Y  L  S  V  V  L  N  Q  L  C  V  L  H  E  K  T  P  V  S  D
AAAGTGGGCAGCAAATGTTGTAAACATCCTGAAGCAAAAAGAATGCCCTGTGCAGAAGACTATCTATCCGTGGTCCTGAACCAGTTATGTGTGTTGCATGAGAAAACGCCAGTAAGTGAC
      1450      1460      1470      1480      1490      1500      1510      1520      1530      1540      1550      1560

R  V  T  K  C  C  T  E  S  L  V  N  R  R  P  C  F  S  A  L  E  V  D  E  T  Y  V  P  K  E  F  N  A  E  T  F  T  F  H  A
AGAGTCACAAAATGCTGCACAGAGTCCTTGGTGAACAGGCGACCATGCTTTTCAGCTCTGGAAGTCGATGAAACATACGTTCCCAAAGAGTTTAATGCTGAAACATTCACCTTCCATGCA
      1570      1580      1590      1600      1610      1620      1630      1640      1650      1660      1670      1680

D  I  C  T  L  S  E  K  E  R  Q  I  K  K  Q  T  A  L  V  E  L  V  K  H  K  P  K  A  T  K  E  Q  L  K  A  V  M  D  D  F
GATATATGCACACTTTCTGAGAAGGAGAGACAAATCAAGAAACAAACTGCACTTGTTGAGCTTGTGAAACACAAGCCCAAGGCAACAAAAGAGCAACTGAAAGCTGTTATGGATGATTTC
      1690      1700      1710      1720      1730      1740      1750      1760      1770      1780      1790      1800

A  A  F  V  E  K  C  C  K  A  D  D  K  E  T  C  F  A  E  E  G  K  K  L  V  A  A  S  Q  A  A  L  G  L  *
GCAGCTTTTGTAGAGAAGTGCTGCAAGGCTGACGATAAGGAGACCTGCTTTGCCGAGGAGGGTAAAAAACTTGTTGCTGCAAGTCAAGCTGCCTTAGGCTTATAACATCTACATTTAAAA
      1810      1820      1830      1840      1850      1860      1870      1880      1890      1900      1910      1920

GCATCTCAGCCTACCATGAGAATAAGAGAAAGAAAATGAAGATCAAAAGCTTATTCATCTGTTTTCTTTTTCGTTGGTGTAAAGCCAACACCCTGTCTAAAAAACATAAATTTCTTTAAT
      1930      1940      1950      1960      1970      1980      1990      2000      2010      2020      2030      2040

CATTTTGCCTCTTTTCTCTGTGCTTCAATTAATAAAAAATGGAAAGAATCTAATAGAGTGGTACAGCACTGTTATTTTTCAAAGATGTGTTGCTATCCTGAAAATTCTGTAGGTTCTGTG
      2050      2060      2070      2080      2090      2100      2110      2120      2130      2140      2150      2160

GAAGTTCCAGTGTTCTCTCTTATTCCACTTCGGTAGAGGATTTCTAGTTTCTGTGGGCTAATTAAATAAATCACTAATACTCTTCTAAGTTAAAAAAA
      2170      2180      2190      2200      2210      2220      2230      2240      2250         2        12        22
```

Fig. 4. 5' Non-coding region modification and signal peptide sequence excision

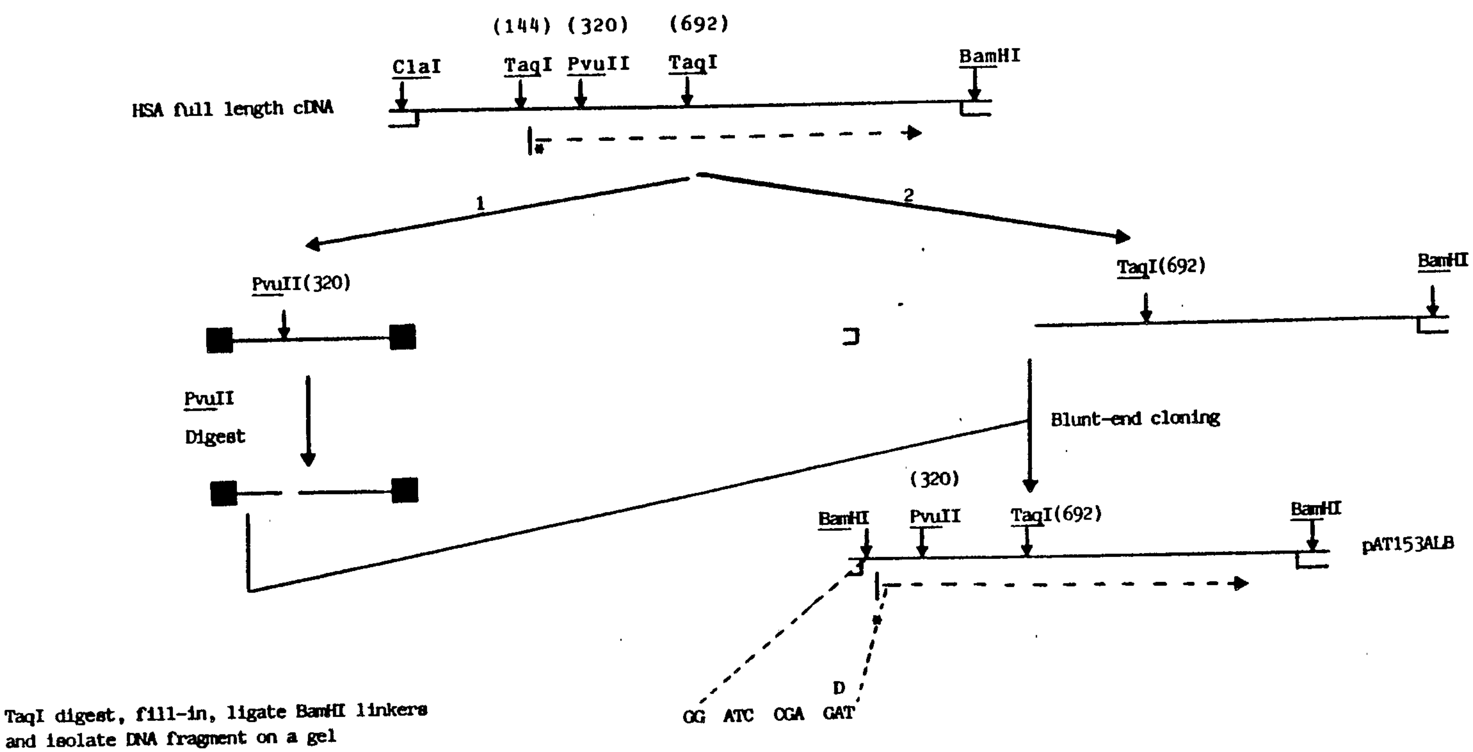


1. TaqI digest, fill-in, ligate BamHI linkers and isolate DNA fragment on a gel
2. PvuII/ClaI double digest, "fill-in"

* Mature HSA

_ cDNA

▭ pAT153 vector DNA

■ BamHI linkers

*Fig.5.* Construction of an "authentic" HSA cDNA (MET-HSA)

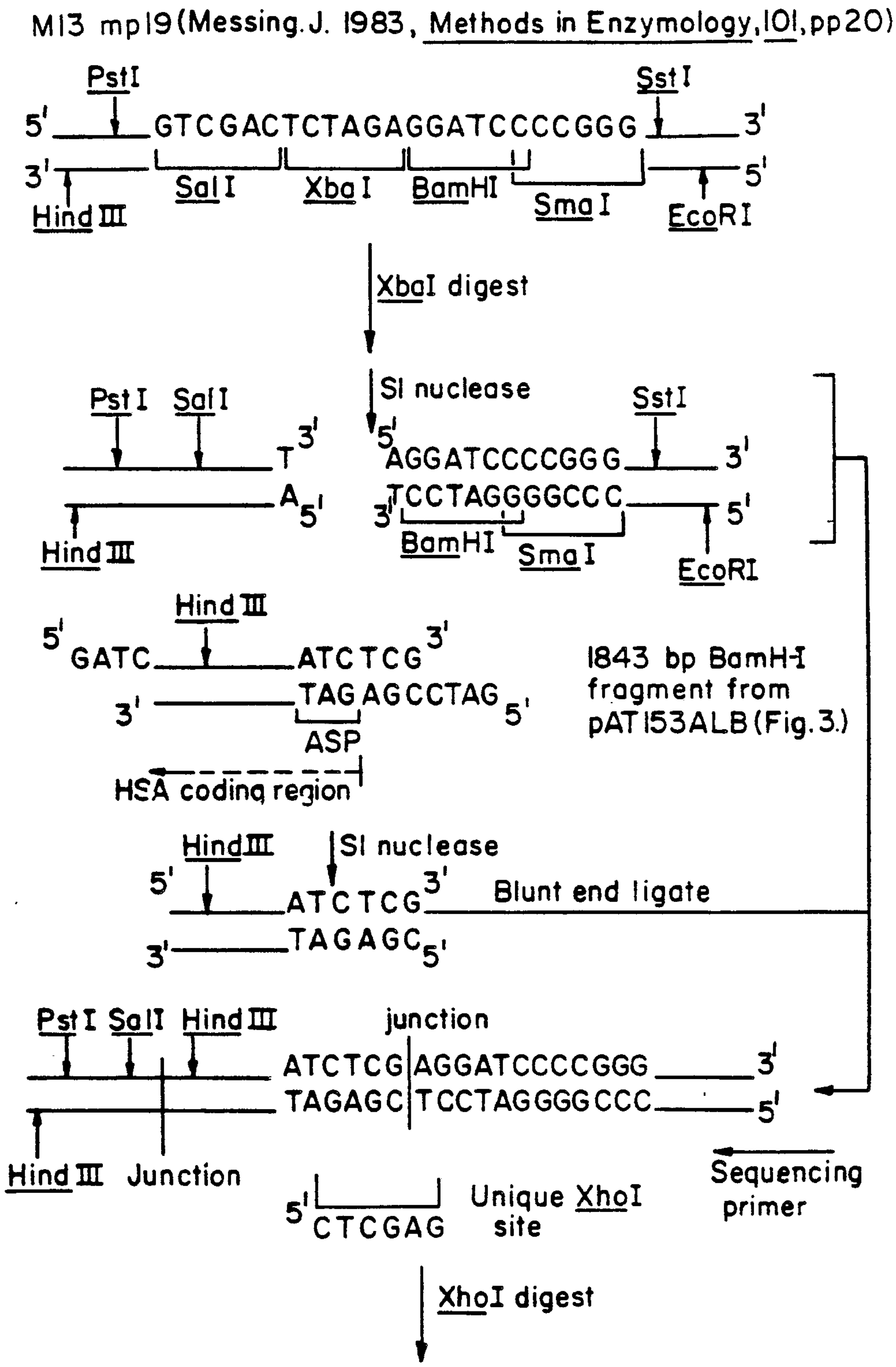

Fig.5(Contd.)

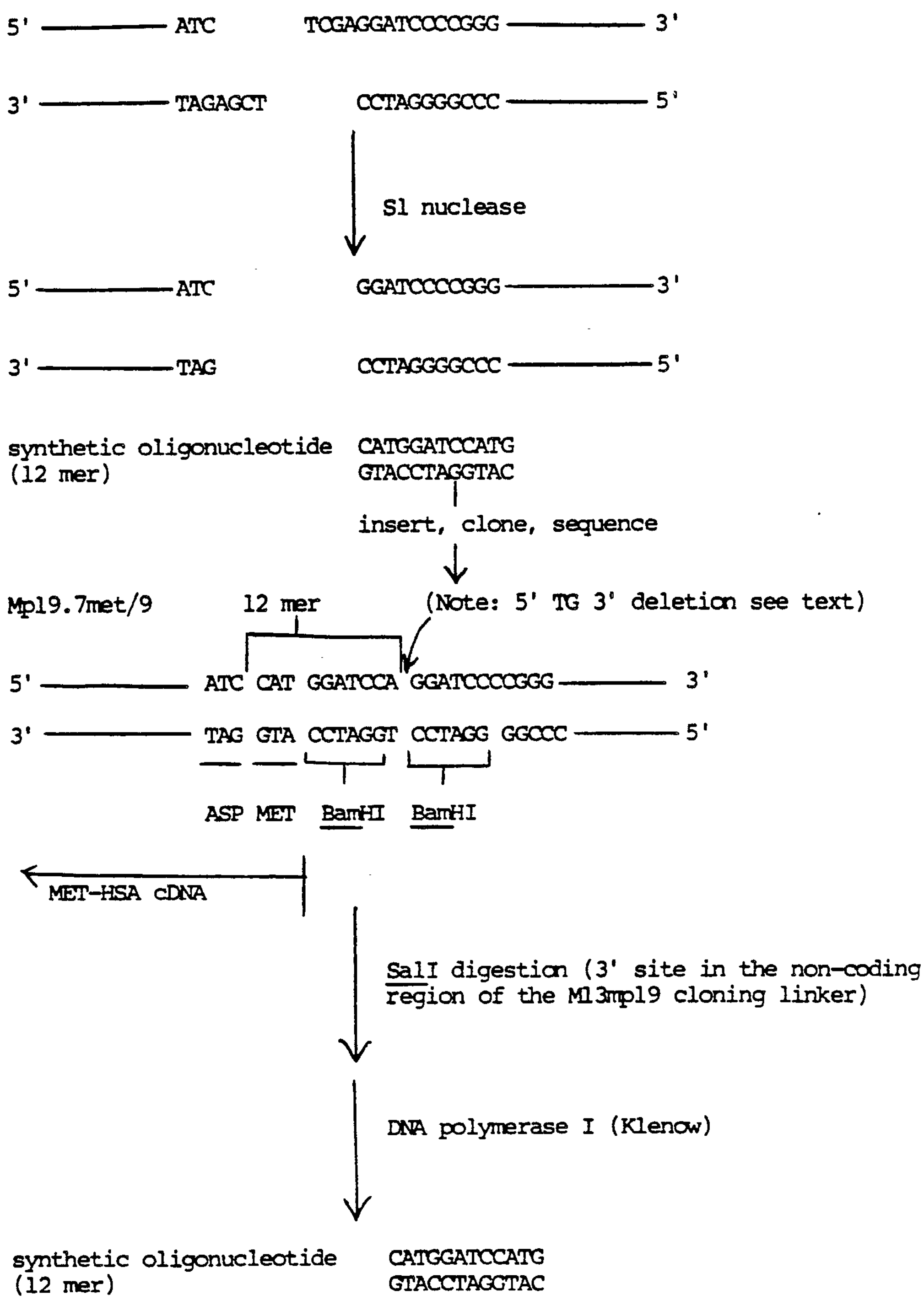
5' ATC TCGAGGATCCCCGGG 3'
3' TAGAGCT CCTAGGGGCCC 5'
S1 nuclease
5' ATC GGATCCCCGGG 3'
3' TAG CCTAGGGGCCC 5'
synthetic oligonucleotide
(12 mer)
CATGGATCCATG
GTACCTAGGTAC
insert, clone, sequence
Mp19.7met/9
12 mer
(Note: 5' TG 3' deletion see text)
5' ATC CAT GGATCCA GGATCCCCGGG 3'
3' TAG GTA CCTAGGT CCTAGG GGCCC 5'
ASP MET BamHI BamHI
MET-HSA cDNA
SalI digestion (3' site in the non-coding region of the M13mp19 cloning linker)
DNA polymerase I (Klenow)
synthetic oligonucleotide
(12 mer)
CATGGATCCATG
GTACCTAGGTAC

Fig.5(Contd.)

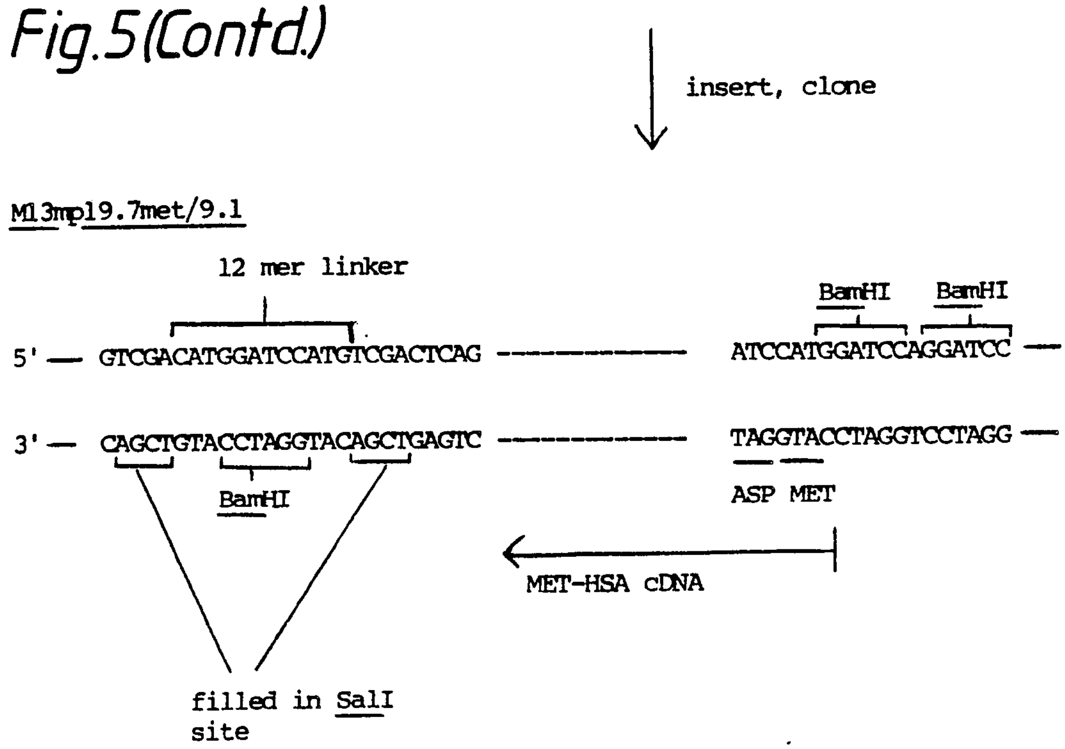
insert, clone
M13mp19.7met/9.1
12 mer linker
BamHI
BamHI
5' — GTCGACATGGATCCATGTCGACTCAG
ATCCATGGATCCAGGATCC —
3' — CAGCTGTACCTAGGTACAGCTGAGTC
TAGGTACCTAGGTCCTAGG —
BamHI
ASP MET
MET-HSA cDNA
filled in SalI site

# Fig 6 Plasmid pEK113

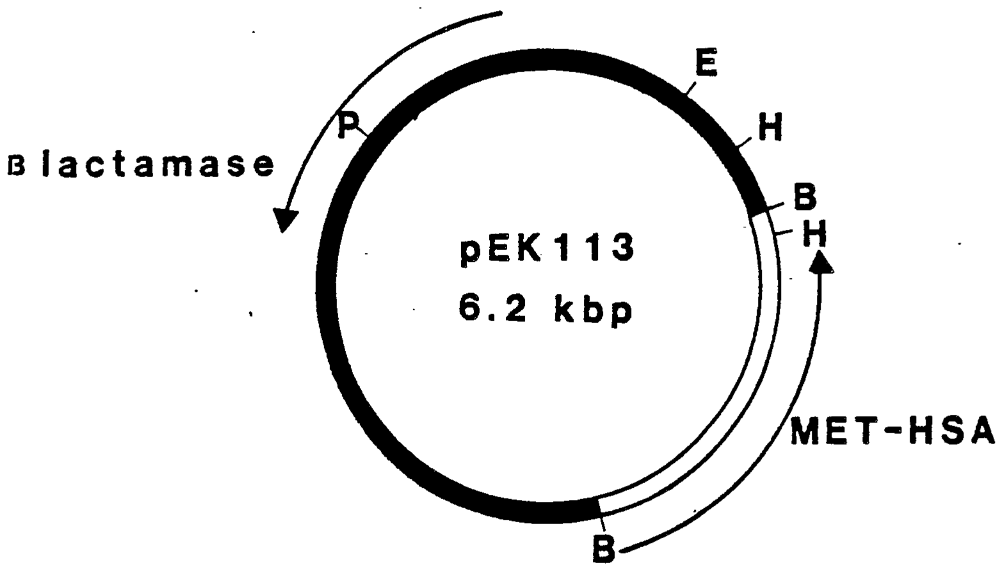


pBR322 DNA
MET-HSA DNA
Restriction endonuclease sites (see Fig. 1)

Fig.7 Construction of plasmid pEHB11 MET-HSA

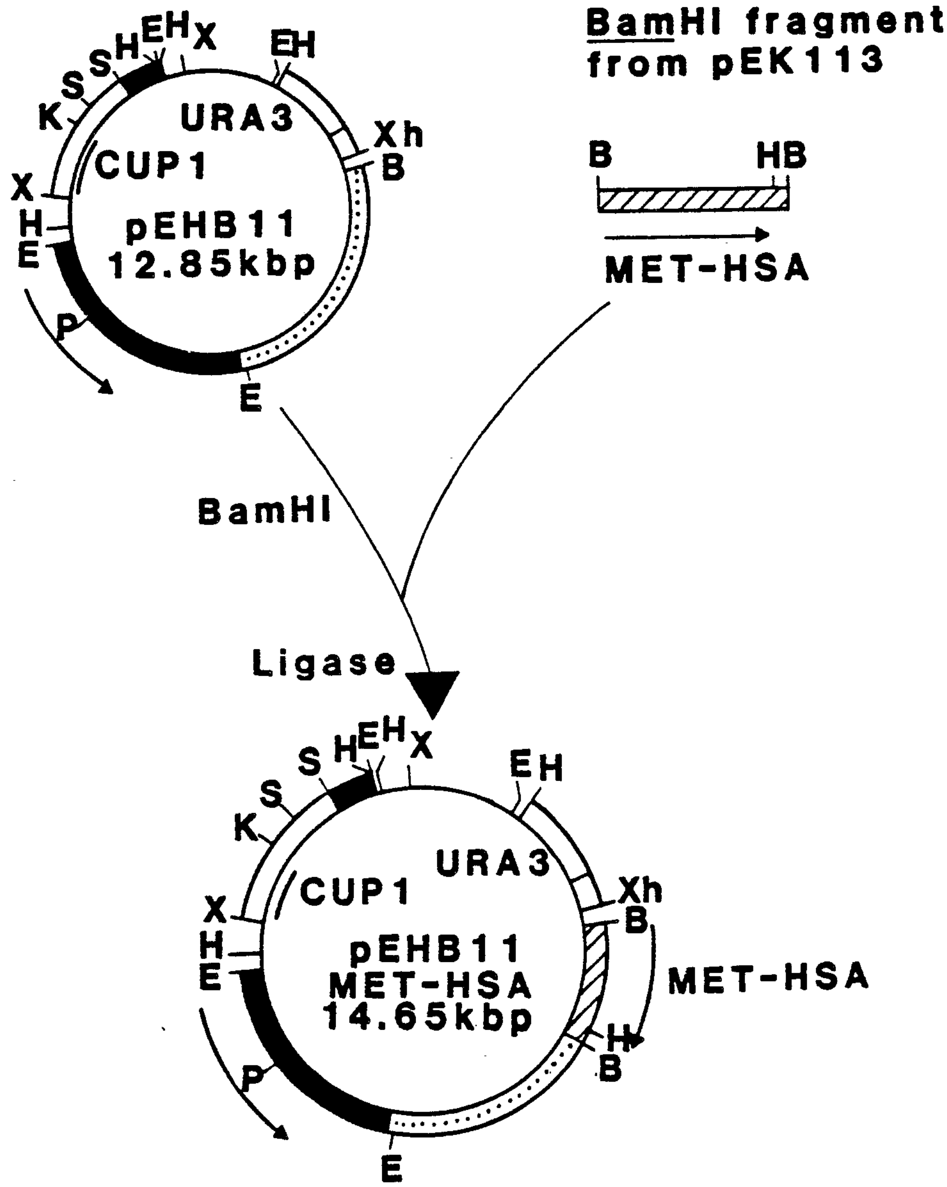

# Fig.8 Construction of plasmid pET13:1-MET-HSA

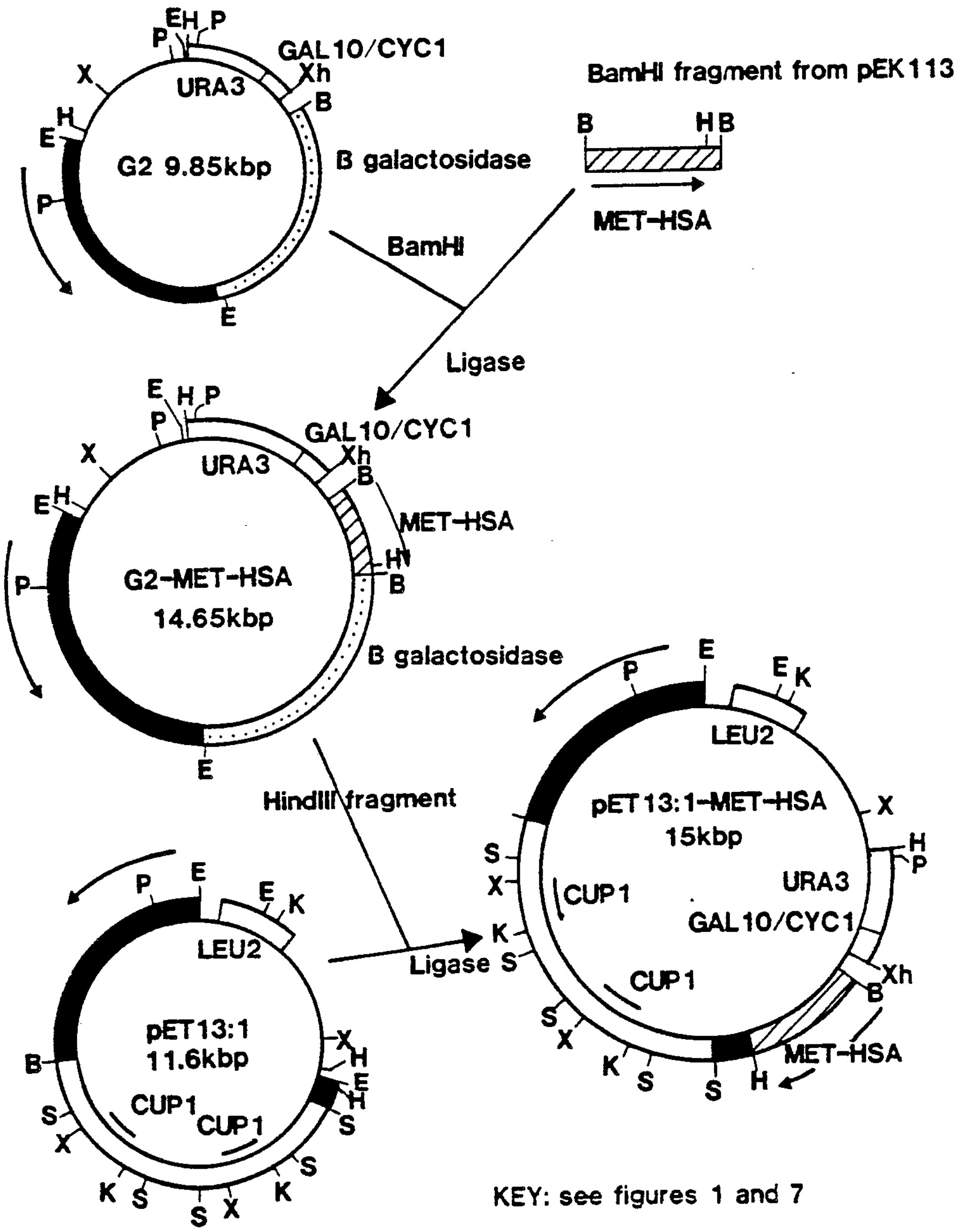


KEY: see figures 1 and 7